# FASCICULE DE BREVET EUROPEEN

(11) **EP 0 900 090 B1**
(45) Date de publication et mention de la délivrance du brevet: **06.02.2002**
(21) Numéro de dépôt: 97921891.4
(22) Date de dépôt: 25.04.1997
(51) Int. Cl.: A61L 9/12, A61L 9/00, A61L 9/01, A61L 9/16

(54) **DISPOSITIF ET PROCEDE DE FILTRATION POUR NEUTRALISER LES MAUVAISES ODEURS**
VORRICHTUNG UND FILTRATIONSVERFAHREN ZUR NEUTRALISIERUNG VON SCHLECHTEN GERÜCHEN
FILTERING DEVICE AND METHOD FOR NEUTRALIZING BAD SMELLS

(30) Priorité: 26.04.1996 FR 9605326
(43) Date de publication de la demande: 10.03.1999
(73) Titulaire: Patent Invest Group Holding SA, 2953 Luxemburg (LU)
(72) Inventeur: BOURGUIGNON, Christiane, F-78240 Aigremont (FR)
(74) Mandataire: Benech, Frédéric
(86) Numéro de dépôt international: FR9700749
(87) Numéro de publication internationale: WO9740863

(56) Documents cités:
- WO-A-86/05120
- DATABASE WPI Section Ch, Week 9329 Derwent Publications Ltd., London, GB; Class D22, AN 93-231547 XP002036165 & JP 05 154 187 A (MITSUBISHI MATERIALS CORP) , 22 Juin 1993

## Description

La présente invention concerne un dispositif de filtration pour neutraliser les mauvaises odeurs volatiles présentes dans une atmosphère ou véhiculées par un courant gazeux.

Elle concerne également un procédé mettant en oeuvre une telle filtration.

Elle trouve une application particulièrement importante bien que non exclusive dans le domaine du traitement des mauvaises odeurs telles que les odeurs corporelles de salle de bain ou de salons de coiffure, les odeurs de cuisine, les odeurs de tabac ainsi que les odeurs particulièrement tenaces comme celles d'excréments ou encore les odeurs de matériaux en décomposition dans les décharges publiques.

Elle est également applicable aux odeurs de cheminée d'usines, de stations d'épuration et plus généralement à tous effluents gazeux malodorants issus de sites industriels, commerciaux ou particuliers.

On connaît déjà différentes méthodes et dispositifs permettant de traiter les mauvaises odeurs.

Une première technique consiste à surmonter ces odeurs par masquage soit quantitativement en parfumant l'atmosphère avec un produit d'arôme plus plaisant en grande quantité, soit par fixation des molécules malodorantes, en général en les complexant avec d'autres molécules, pour donner un produit d'un arôme différent et plus agréable.

Cette technique de masquage présente cependant des inconvénients.

En effet, une telle surimposition ne permet pas d'éliminer complètement les mauvaises odeurs, compte tenu notamment de la très grande sensibilité des nerfs olfactifs humains.

Par exemple l'homme reste sensible au sulfure d'hydrogène alors que la proportion de ce dernier dans l'atmosphère ne dépasse pas 3ppm.

Une autre technologie envisagée est celle de la filtration.

Elle consiste à réduire les mauvaises odeurs en filtrant les molécules odorantes à l'émission.

On connaît ainsi des filtres à charbons actifs qui permettent des résultats acceptables. De tels filtres ne pouvant être absolus dans la pratique, laissent cependant passer des molécules malodorantes.

La présente invention vise à fournir un dispositif et un procédé de traitement des mauvaises odeurs répondant mieux que ceux antérieurement connus aux exigences de la pratique notamment en ce qu'elle permet une meilleure épuration de l'air de façon simple, efficace et peu coûteuse.

En mettant en oeuvre en combinaison, une filtration mécanique, avec adsorption des molécules malodorantes, et un relargage de molécules parfumantes et/ou d'une composition de ces molécules d'odeurs agréables, l'atmosphère ou le courant gazeux malodorant traité sont ainsi apurés et agréablement parfumés.

Dans ce but l'invention propose essentiellement un dispositif de filtration pour neutraliser les mauvaises odeurs volatiles présentes dans une atmosphère ou véhiculées par un courant gazeux, caractérisé en ce qu'il comporte au moins une couche de matériau en zéolite naturel d'épaisseur déterminée, ladite couche étant imprégnée d'une solution aqueuse, avec pour 100 parties en poids de zéolite, environ 5 à environ 20 parties en poids de ladite solution aqueuse, ladite solution aqueuse comportant pour 1000 parties en poids d'eau, entre environ 1 partie et environ 20 parties en poids d'un mélange composite comprenant, pour une partie d'ions ammonium. ou équivalent, une partie d'au moins une composition de nature parfumante et/ou désodorisante.

L'épaisseur de la couche est déterminée d'une façon à la portée de l'homme du métier, en fonction de l'utilisation spécifique du filtre dépendant des caractéristiques du gaz filtré, c'est-à-dire essentiellement du débit moyen des molécules malodorantes passant au travers du filtre.

La nature adsorbante de la zéolite naturelle, l'humidification de l'atmosphère réalisée grâce à ce filtre imprégné d'eau de façon appropriée et renouvelée avec le temps, ainsi que les possibilités de relargage de molécules parfumantes et/ou désodorisantes dans le courant gazeux ou l'atmosphère filtrée autorisent ainsi une excellente épuration physico-chimique.

Dans des modes de réalisation avantageux, on a de plus recours à l'une et/ou à l'autre des dispositions suivantes :
- la solution aqueuse est cationique;
- la solution aqueuse comporte entre environ 3 parties et environ 10 parties de mélange composite;
- la couche en zéolite naturel est formée de matériau granuleux, de granulométrie moyenne comprise entre de l'ordre de 0,1 cm et de l'ordre de 5 cm;
- la composition de nature parfumante et/ou désodorisante comprend une quantité efficace pour neutraliser les mauvaises odeurs d'un solvant représenté par la formule développée ci-après : 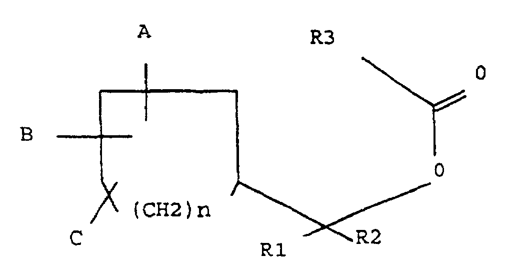
où n est un nombre entier valant 2 à 4, A, B et C représentent chacun indépendamment l'hydrogène, un groupe alkyle inférieur ayant 1 à 5 atomes de carbone ou un groupe alkényle inférieur ayant 3 à 5 atomes de carbone, à condition que lorsque deux des groupes, A, B et C sont des groupes alkyle ou alcényle, ils soient fixés à des atomes de carbone différents et pourvu que la somme des atomes de carbone dans A, B et C ne soit pas supérieure à 7, R1 et R2 représentent chacun indépendamment l'hydrogène ou un groupe alkyle inférieur ayant 1 à 5 atomes de carbone, où R1 et R2 pris ensemble représentent CH2m où m est un nombre entier valant 2 à 6, R3 représente l'hydrogène ou un groupe alkyle ou alkényle inférieur ayant jusqu'à 6 atomes de carbone, pourvu que la somme du plus grand nombre d'atomes de carbone dans R1 ou R2 plus R3 ne soit pas supérieure à 10.

De tels composés utilisés sous forme de solvant, présentent des molécules pour parties hydrophiles et pour parties hydrophobes qui ont montré de façon inattendue, lors des tests réalisés, qu'elles amélioraient le mécanisme d'imprégnation de la zéolite et de relargage de la composition parfumante et/ou désodorisante;
- la composition de nature parfumante et/ou désodorisante est élaborée à partir d'esters aromatiques et/ou de formation complexe d'aldéhyde;
- la composition de nature parfumante comporte un premier aldéhyde choisi parmi les aldéhydes aliphatiques acycliques et non terpéniques, les aldéhydes alicycliques non terpéniques, les aldéhydes terpéniques, les aldéhydes aliphatiques substitués par un groupe aromatique, les aldéhydes biofonctionnels et/ou un second aldéhyde choisi parmi les aldéhydes possédant l'insaturation en α de la fonction aldéhyde conjuguée avec un cycle aromatique et les aldéhydes dont la fonction est portée par un cycle aromatique;
- la zéolite naturelle comprend au moins un type de zéolite choisie parmi la clinoptilotite, la mordenite, la chabazite, l'érionite, la phillipsite, l'heulandite, l'analcime, et/ou en provenance de tous gisements lithique de matériau de type zéolite comportant des caractéristiques physico-chimiques présentant des qualités équivalentes aux matériaux cités ci-avant, d'adsorption, de catalyse et d'échange.
- le dispositif comporte des moyens de circulation forcée du courant gazeux au travers de la couche de matériau du dispositif.

L'invention propose également un procédé de traitement des mauvaises odeurs volatiles présentes dans une atmosphère ou véhiculées par un courant gazeux, caractérisé en ce que on traite l'atmosphère ou le courant gazeux par filtration avec un filtre composé d'une couche de matériau en zéolite naturel d'épaisseur déterminée imprégnée d'une solution aqueuse, avec pour 100 parties en poids de zéolite, environ 5 à environ 20 parties en poids de ladite solution aqueuse, ladite solution aqueuse comportant, pour 1000 parties en poids d'eau, entre environ 1 partie et environ 20 parties en poids d'un mélange composite comprenant, pour une partie d'ions ammonium ou équivalent, une partie d'au moins une composition de nature parfumante et/ou désodorisante.

Avantageusement, on réalise une circulation forcée de l'atmosphère ou du courant gazeux au travers du filtre par aspiration de l'atmosphère ou du courant gazeux au travers du filtre.

L'invention sera mieux comprise à la lecture de la description qui suit de modes particuliers de réalisation donné à titre d'exemple non limitatif.
- La figure 1 montre schématiquement un mode de réalisation du dispositif selon l'invention disposé au-dessus d'un courant gazeux à désodoriser.
- La figure 2 est une vue schématique d'une couche de matériau en zéolite selon l'invention. La structure est par exemple métallique, peinte, de forme extérieure sensiblement cylindrique.

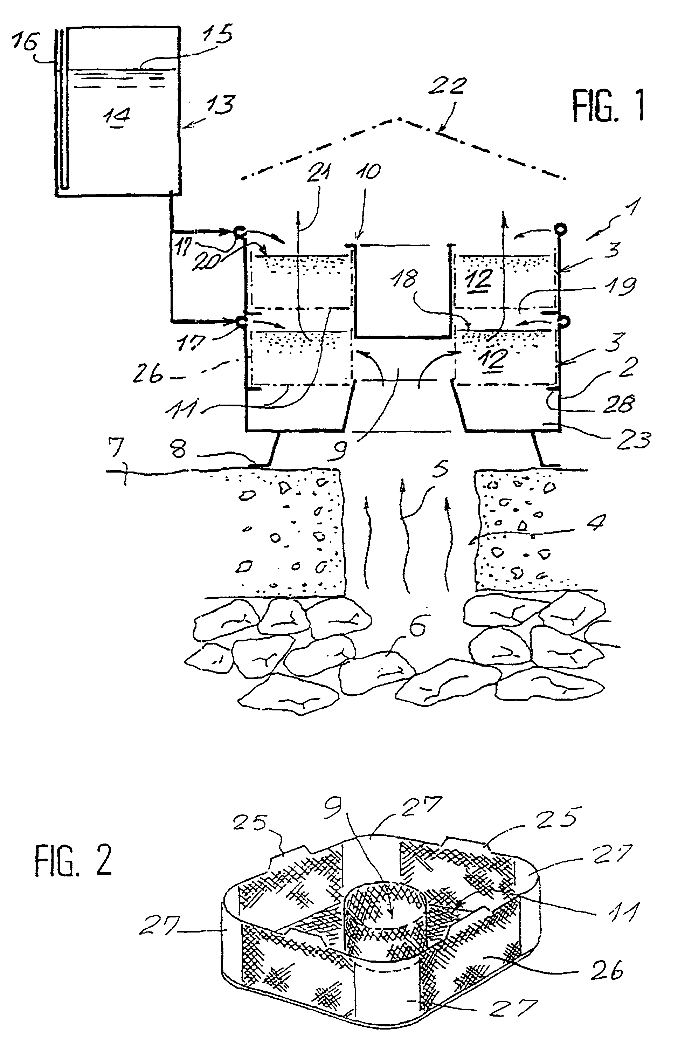

La figure 1 montre schématiquement un dispositif filtrant ou filtre 1, amovible, comprenant une structure 2 de support de deux couches annulaires 3, horizontales, par exemple identiques, en matériau granuleux à base de zéolite selon l'invention.

Les couches sont d'épaisseur déterminée, par exemple chacune de 5 cm d'épaisseur.

Le filtre 1 est placé en dessus d'un puits 4 d'évacuation de gaz 5 de décomposition chargés en mauvaises odeurs issues d'un stockage de déchets pourrissants 6, par ailleurs recouverts d'une couche de terre 7.

La dimension externe du filtre et le diamètre externe des couches annulaires est fonction de la largeur du puits 4.

La structure 2 de support repose sur le rebord du puits via un support conique 8 permettant la canalisation approximativement étanche des gaz, qui s'élèvent ici par extraction naturelle, et leur passage par le filtre.

Celui-ci comporte une cheminée centrale 9, cylindrique en partie haute, fermée par exemple à mi-hauteur du filtre, avec une embase conique en partie basse permettant de guider les gaz.

La cheminée 9 est ajourée ou grillagée sur les parois latérales 10 de sa partie haute cylindrique de façon à permettre le passage des gaz radialement vers l'extérieur et au travers des parois de ladite cheminée, pour pénétrer dans la couche inférieure 3 du filtre, qu'elle traverse ensuite verticalement, ainsi que la couche 3 supérieure.

Chaque couche (voir figure 2) repose sur un tamis en forme de rondelle métallique 11, de soutien gravitaire du matériau 12, granulaire, compacté et humidifié avec un mélange composite selon l'invention.

L'humidification des couches de zéolite se fait par exemple via un goutte à goutte à écoulement gravitaire, à partir d'un réservoir 13 de stockage du mélange composite 14 dont le niveau 15 est régulièrement contrôlé par exemple via un indicateur visuel 16.

Plus précisément le réservoir 13 alimente par exemple deux rampes 17 à buses d'aspersion régulièrement reparties, chacune desdites rampe étant située à la périphérie ou encore de façon centrée, au dessus de la surface annulaire supérieure d'une couche correspondante.

Pour permettre l'installation de la rampe de la couche inférieure, la surface supérieure 18 de celle-ci débouche dans un espace annulaire 19, fermé sur ses parois, séparant les deux couches.

La surface supérieure 20 de la couche supérieure débouche quant à elle à l'air libre pour libérer les gaz épurés et/ou parfumés 21.

Un toit 22 de protection contre les intempéries, par exemple en forme de chapeau chinois schématisé en trait mixte sur la figure 1, est par ailleurs prévu.

Enfin le fond de la structure 2 est formé par une cuve annulaire 23, de rétention du mélange composite qui n'aurait pas été absorbé par la zéolite. Elle est délimitée d'un côté par l'embase conique inférieure de la cheminée 9 et de l'autre côté par la paroi externe de la structure.

En cas de trop plein, le mélange composite vient imprégner la face inférieure de la couche inférieure 3, et se déversera par trop plein dans la cheminée 9 vers le puits 4.

Le filtre 1 est entièrement démontable, par exemple par le haut, ce qui permet ainsi le remplacement des couches 3 de zéolite.

Pour se faire, il est prévu des poignées 25 de préhension des couches 3 chacune enserrée dans une enveloppe 26 de cohésion à coins renforcés 27.

L'enveloppe 26 est quant à elle agencée pour venir reposer sur des butées 28 fixées sur les parois internes de la structure 2.

Il en ressort une grande simplicité, une robustesse, et une fiabilité de mise en oeuvre de maintenance du filtre particulièrement remarquable.

De façon générale, concernant le mélange composite, on citera comme composés pouvant convenir selon l'invention, les composés choisis parmi les composés suivants :
- les alcools aliphatiques, avantageusement de C10 à C12, comme le décanol, le citronéllol, le géraniol;
- les aldéhydes, avantageusement de C10 à C13, soit aliphatiques comme le dodécanal normal ou ramifié, l'aldéhyde myrac, soit aromatiques comme l'aldéhyde cyclamen, l'hélional, l'héliotropine, le paraméthylphényl-acétaldéhyde, la vanilline et ses dérivés;
- les cétones aliphatiques, avantageusement en C13 et C14, comme les alpha et béta ionones et damascones ainsi que les cétones aliphatiques et aromatiques à odeur musquée pouvant avoir jusqu'à 18 atomes de carbone;
- les esters aliphatiques, avantageusement en C8 et C15, comme le dihydrojasmonate de méthyle, le jasmonate de méthyle, le cinnamate de méthyle, le méthylphénylglycidate d'éthyle, les esters aromatiques comme l'anthranilate de méthyle, le N-méthyl-anthranilate de méthyle, le phénylacétate de p-crésyle, le salicylate d'amyle;
- les lactones aromatiques comme la coumarine, la dihydrocoumarine et les lactones aliphatiques comme la gamma-décalactone, dodécalactone et undécalactone;
- les phénols comme l'eugénol et l'isoeugénol;
- les éthers aromatiques comme l'oxyde de diphényle, les éthers méthylés et éthylés du naphtol, le galaxolide;
- les composés azotés du type amines comme l'indole et ses composés de réaction avec l'hydroxycitronellal dénommés indolène et les nitriles aliphatiques comme le tridécène-2-nitrile;
- les amines aromatiques y compris les dérivés pyridiniques comme la (méthyl-2'-pentène-2'-yl)-2-méthyl-5-pyridine.

Parmi les composés mentionnés ci-dessus convenant selon l'invention, on citera de préférence la coumarine, l'allyl-ionone, l'eugénol et l'isoeugénol, le dihydrojasmonate de méthyle, l'indole et l'indolène, l'hélional, l'alpha et la béta ionone et la (méthyl-2'-pentène-2'-yl)-2-méthyl-5-pyridine.

Ceux des composés qui sont liquides peuvent être utilisés tels quels. Par contre, les composés cristallisés seront utilisés après leur dissolution dans leur solvant habituel bien connu en parfumerie comme le phtalate d'éthyle, le benzoate de benzyle, le citrate d'éthyle et analogues.

En outre, si on le désire, les composés selon l'invention peuvent aussi être utilisés en mélanges dans des proportions variables donnant une formule n'ayant pratiquement aucun effet olfactif désagréable.

Rien n'empêche toutefois d'ajouter aux composés selon l'invention, pris seuls ou en mélanges, et à volonté d'autres matières premières parfumantes agrémentant pour l'usager et pour l'environnement l'effet olfactif de la formulation à appliquer.

Pour mieux illustrer les résultats avantageux obtenus avec le procédé selon l'invention il est donné ci-après deux exemples de façon nullement limitative.

Le test employé est effectué avec un panel d'au minimum cinquante personnes, dont cinq au moins sont des spécialistes de l'olfaction, les autres étant sélectionnées sans critère particulier.

L'effluent à traiter est quant à lui défini de la façon suivante :

Pour un effluent quantifiable et isolé, par exemple H2S :
- Par réduction physique :
   En mesurant sa concentration dans l'air avant traitement et sa concentration dans l'air filtré,
   - Par réduction sensitive :

Pour ce faire on fait noter sur une échelle d'intensité par l'ensemble du panel les sensations perçues en inspiration par le biais d'un olfactomètre ou d'un test en bêcher :
- de l 'air traité par le système de filtration et imprégné de solution désodorisante,
- de deux échantillons "leurre", l'un parfumé et l'autre malodorant,
- d'un air chargé artificiellement d'une concentration de H2S identique à celle de l'air traité, sans solution désodorisante.

Dans le cas d'un effluent malodorant de type complexe, par exemple provenant d'une dégradation organique, on fait noter sur une échelle d'intensité par l'ensemble du panel les sensations perçues en inspiration par le biais d'un olfactomètre ou d'un test en bêcher :
- de l'air traité par le système de filtration et imprégné de solution désodorisante
- de deux échantillons "leurre", l'un parfumé et l'autre malodorant d'une autre nature,
- d'un échantillon d'air non parfumé chargé de H²S, à la concentration résiduelle relevée d'un air traité.

Dans tous les cas, les panelistes notent leurs sensations sur deux échelles graduées (A et B) en distinguant l'intensité de la mauvaise odeur (échelle - A -) et l'intensité totale de l'odeur (échelle - B -).

Compte tenu de l'anosmie temporaire qui est induite par cette technologie, il est recommandé d'attendre au moins une minute entre deux échantillons. Cette attente peut être comblée, soit par une question de contrôle, soit en sentant ou en gouttant un produit tout à fait différent (eau de toilette, chewing-gum).

L'odeur d'origine reçoit sur les échelles graduées la valeur 0.

Pour chaque échelle, la graduation va, par pas de 5, de -20 à +20, la note -20 représentant la neutralité absolue et l'absence de toute sensation olfactive.

Les résultats sont obtenus en faisant la moyenne, pour chaque échelle, des notes attribuées par les panélistes.

### Echelle A :

Une moyenne inférieure à 0 reflète une réduction de la mauvaise odeur, une moyenne supérieure à 0 reflète un accroissement de la mauvaise odeur.

### Echelle B :

Une moyenne inférieure à 0 reflète une réduction de l'intensité de l'odeur perçue, une moyenne supérieure à 0 reflète un accroissement de l'intensité de l'odeur perçue.

Il est ensuite procédé à l'analyse des résultats selon les critères suivants :
- Une moyenne A supérieure à 0, avec une moyenne supérieure à 0 révèle un effet de Masquage,
- Une moyenne A inférieure à 0, avec une moyenne supérieure à 0 révèle un effet d'inhibition-réduction.

Compte tenu des traces résiduelles d'effluents malodorants et de molécules parfumantes, on considère que le résultat optimal se situe dans la fourchette -5/-10 des échelles A et B.

### TEST 1 :

Air chargé de 40ppm de H2S à 20°C
Filtre Zéolite Granulats de 0.8/1.2 cm
Clinoptinolite
Epaisseur : 40 mm
   Imbibé à 15% en poids d'une solution aqueuse composite à base d'ions ammoniums, esters, aldéhydes, et solvants du type connu sous la dénomination VAILEX commercialisée par la Société BUSH BOAKE ALLEN, à une concentration de 5 pour 1.000.
Vitesse de passage de l'Air : 0,04 m/seconde

### Résultats observés :

a) Mesure de la concentration H2S après filtration : 7ppm
b) odeur résiduelle - Echantillon témoin : Air + H²S 7 ppm, correspondant à la valeur 0 des échelles A & B
panélisation de l'odeur résiduelle : Echelle A : - 6 et Echelle B : -5

### TEST N°2 :

Odeur forte de décomposition organique (prélèvement sur décharge)

Présence de Bio-gaz, méthane, mercaptans, H²S, NH3, ...
Filtre Zéolite Granulats de 0.8/1.2 cm
Clinoptilite 50% - Chabazite 50%
Epaisseur : 30 mm

Imbibé à 15% en poids d'une solution aqueuse composite à base d'ions ammoniums, esters, aldéhydes, et solvants du type connu sous la dénomination VAILEX, à une concentration de 4 pour 1.000.
Vitesse de passage de l'Air : 0,1 m/seconde
Panélisation de l'odeur résiduelle : Echelle A : - 8 et Echelle B : -6.

Comme il va de soi et comme il résulte de ce qui précède, la présente invention ne se limite pas au mode de réalisation plus particulièrement décrit. Elle en embrasse au contraire toutes les variantes et notamment celles où le filtre comporte des moyens engendrant une circulation forcée, celles où il ne comporte qu'une couche ou plusieurs couches de matériau à base de zéolite, celles où le système d'humidification est différent.

## Revendications

1. Dispositif (1) de filtration pour neutraliser les mauvaises odeurs volatiles présentes dans une atmosphère ou véhiculées par un courant gazeux (5), comportant au moins une couche (3) de matériau (12) adsorbant d'épaisseur déterminée imprégné d'une solution aqueuse (14) avec pour 100 parties en poids de matériau, environ 5 à 20 parties en poids de ladite solution aqueuse, **caractérisé en ce que**
le matériau est de la zéolithe naturelle comprenant au moins un matériau choisi parmi la clinoptilolite, la mordenite, la chabazite, l'érionite, la phillipsite, l'heulandite et l'analcime,
**en ce que** le dispositif comporte des moyens d'humidification
et **en ce que** ladite solution aqueuse est cationique et comporte pour 1000 parties en poids d'eau, entre environ 1 et 20 parties en poids d'un mélange composite comprenant, pour une partie d'ions ammonium ou équivalent, une partie d'au moins une composition de nature parfumante et/ou désodorisante.

2. Dispositif selon la revendication 1, **caractérisée en ce que** la solution aqueuse comporte entre environ 3 et 10 parties de mélange composite.

3. Dispositif selon l'une quelconque des revendications précédentes, **caractérisé en ce que** la couche (3) en zéolite naturel est formée de matériau granuleux de granulométrie moyenne comprise entre de l'ordre de 0,1 cm et de l'ordre de 5 cm.

4. Dispositif selon l'une quelconque des revendications précédentes, **caractérisé en ce que** la composition de nature parfumante et/ou désodorisante comprend une quantité efficace pour neutraliser les mauvaises odeurs d'un composé représenté par la formule développée ci-après : 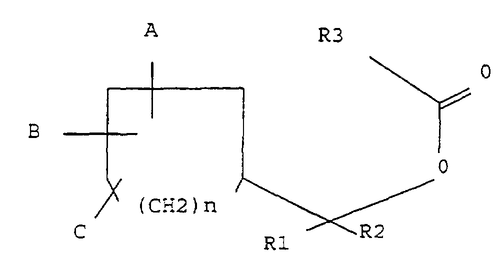 où n est un nombre entier valant 2 à 4, A, B et C représentent chacun indépendamment l'hydrogène, un groupe alkyle inférieur ayant 1 à 5 atomes de carbone ou un groupe alkényle inférieur ayant 3 à 5 atomes de carbone, à condition que lorsque deux des groupes, A, B et C sont des groupes alkyle ou alcényle, ils soient fixés à des atomes de carbone différents et pourvu que la somme des atomes de carbone dans A, B et C ne soit pas supérieure à 7, R1 et R2 représentent chacun indépendamment l'hydrogène ou un groupe alkyle inférieur ayant 1 à 5 atomes de carbone, où R1 et R2 pris ensemble représentent CH2m où m est un nombre entier valant 2 à 6, R3 représente l'hydrogène ou un groupe alkyle ou alkényle inférieur ayant jusqu'à 6 atomes de carbone, pourvu que la somme du plus grand nombre d'atomes de carbone dans R1 ou R2 plus R3 ne soit pas supérieure à 10.

5. Dispositif selon l'une quelconque des revendications 1 à 3, **caractérisé en ce que** la composition de nature parfumante et/ou désodorisante est élaborée à partir d'esters aromatiques et/ou de formation complexe d'aldéhydes.

6. Dispositif selon la revendication 5, **caractérisé en ce que** la composition parfumante comporte un premier aldéhyde choisi parmi les aldéhydes aliphatiques acycliques et non terpéniques, les aldéhydes alicycliques non terpéniques, les aldéhydes terpéniques, les aldéhydes aliphatiques substitués par un groupe aromatique, les aldéhydes biofonctionnels et un second aldéhyde choisi parmi les aldéhydes possédant l'insaturation en α de la fonction aldéhyde conjuguée avec un cycle aromatique et les aldéhydes dont la fonction est portée par un cycle aromatique.

7. Dispositif selon l'une quelconque des revendications précédentes, **caractérisé en ce que** il comporte de plus des moyens de circulation forcée du courant gazeux au travers de la couche de matériau du dispositif.

8. Procédé de traitement des mauvaises odeurs volatiles présentes dans une atmosphère ou véhiculées par un courant gazeux, dans lequel on traite l'atmosphère ou le courant gazeux par filtration avec un filtre composé d'une couche de matériau adsorbant d'épaisseur déterminée imprégnée d'une solution aqueuse, avec pour 100 parties en poids de matériau, environ 5 à environ 20 parties en poids de ladite solution aqueuse, **caractérisé en ce que** le matériau est de la zéolithe naturelle comprenant au moins un matériau choisi parmi la clinoptilotite, la mordenite, la chabazite, l'érionite, la phillipsite, l'heulandite et l'analcime.
ladite solution aqueuse comportant pour 1 000 parties en poids d'eau, entre environ 1 partie et environ 20 parties en poids d'un mélange composite comprenant, pour une partie d'ions ammonium ou équivalent, une partie d'au moins une composition de nature parfumante et/ou désodorisante.

9. Procédé selon la revendication 8, **caractérisé en ce que** on réalise une circulation forcée de l'atmosphère ou du courant gazeux au travers du filtre par aspiration de ladite atmosphère ou dudit courant gazeux au travers dudit filtre.

## Patentansprüche

1. Filtrationsvorrichtung (1) für eine Neutralisierung von schlechten flüchtigen Gerüchen, die in einer Atmosphäre anwesend sind oder durch einen Gasstrom (5) übertragen werden, mit wenigstens einer Schicht (3) eines adsorbierenden Materials (12) mit einer vorbestimmten Dicke, die mit einer wässerigen Lösung (14) mit ungefähr 5 bis 20 Gewichtsanteilen der wässerigen Lösung auf je 100 Gewichtsanteile des Materials imprägniert ist,
**dadurch gekennzeichnet,**
**dass** das Material ein natürlicher Zeolith ist, der wenigstens ein Material enthält, das aus Clinoptilotit, Mordenit, Chabazit, Erionit, Phillipsit, Heulandit und Analcim ausgewählt ist,
**dass** die Vorrichtung Mittel zur Befeuchtung enthält
und **dass** die wässerige Lösung kationisch ist und auf 1000 Gewichtsanteilen Wasser zwischen ungefähr 1 und 20 Gewichtsanteile einer Verbundmischung enthält, die auf einen Teil Ammonium-lonen oder eines Äquivalents einen Teil wenigstens einer Zusammensetzung vom parfümierenden und/oder desodorierenden Typ enthält.

2. Vorrichtung nach Anspruch 1,
**dadurch gekennzeichnet,**
**dass** die wässerige Lösung zwischen ungefähr 3 und 10 Teilen der Verbundmischung enthält.

3. Vorrichtung nach einem der vorangehenden Ansprüche,
**dadurch gekennzeichnet,**
**dass** die Schicht (3) aus natürlichem Zeolith aus einem Granulat mit einer mittleren Partikelgröße zwischen ungefähr 0,1 cm und ungefähr 5 cm gebildet ist.

4. Vorrichtung nach einem der vorangehenden Ansprüche,
**dadurch gekennzeichnet,**
**dass** die Zusammensetzung vom parfümierenden und/oder desodorierenden Typ eine Zusammensetzung in einer Menge enthält, die zur Neutralisierung der schlechten Gerüche wirksam ist, und die dargestellt ist durch die folgende Strukturformel: 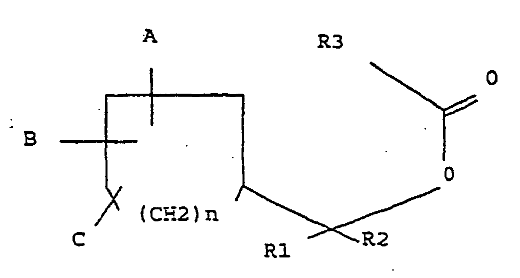 wobei n eine ganze Zahl von 2 bis 4 ist, A, B und C jeweils unabhängig voneinander Wasserstoff, eine niedrige Alkylgruppe mit 1 bis 5 Kohlenstoffatomen oder eine niedrige Alkenylgruppe mit 3 bis 5 Kohlenstoffatomen bedeuten, unter der Bedingung, dass dann, wenn zwei der Gruppen A, B und C Alkyl- oder Alkenyl-Gruppen sind, diese an verschiedene Kohlenstoffatome gebunden sind, und vorausgesetzt, dass die Summe der Kohlenstoffatome in A, B und C nicht größer als 7 ist, R1 und R2 jeweils unabhängig voneinander Wasserstoff oder eine niedrigere Alkylgruppe mit 1 bis 5 Kohlenstoffatomen darstellen, wobei R1 und R2 zusammen CH2m darstellen, wobei m eine ganze Zahl von 2 bis 6 ist, R3 Wasserstoff oder eine niedrige Alkyl- oder Alkenyl-Gruppe mit bis zu 6 Kohlenstoffatomen darstellt, vorausgesetzt, dass die Summe der größten Anzahl von Kohlenstoffatomen in R1 oder R2 plus R3 nicht größer als 10 ist.

5. Vorrichtung nach einem der Ansprüche 1 bis 3,
**dadurch gekennzeichnet,**
**dass** die Zusammensetzung vom parfümierenden und/oder der desodorierenden Typ aus aromatischen Estem und/oder aus Komplexen von Aldehyden gebildet ist.

6. Vorrichtung nach Anspruch 5,
**dadurch gekennzeichnet,**
**dass** die Parfümierungs-Zusammensetzung einen ersten Aldehyd enthält, der ausgewählt ist aus den azyklischen und nicht-terpenischen aliphatischen Aldehyden, den alizyklischen nicht-terpenischen Aldehyden, den terpenischen Aldehyden, den aliphatischen Aldehyden, die mit einer aromatischen Gruppe substituiert sind, den bifunktionalen Aldehyden, und
einen zweiten Aldehyd ausgewählt aus den Aldehyden, die die Ungesättigtheit in α-Stellung zu der Aldehydfunktion in Konjugation zu einem aromatischen Ring besitzen, und aus den Aldehyden, deren Funktion an einem aromatischen Ring sitzt.

7. Vorrichtung nach einem der vorangehenden Ansprüche,
**dadurch gekennzeichnet,**
**dass** sie außerdem Mittel zum Zwangsumlauf des Gasstroms durch die Schicht des Materials in der Vorrichtung enthält.

8. Verfahren zur Behandlung schlechter, flüchtiger Gerüche, die in einer Atmosphäre anwesend sind oder durch einen Gasstrom übertragen werden, wobei die Atmosphäre oder der Gasstrom durch eine Filtration mit einem Filter aus einer Schicht mit einem adsorbierenden Material mit vorbestimmter Dicke behandelt werden, das mit einer wässerigen Lösung mit ungefähr 5 bis ungefähr 20 Gewichtsanteilen an wässeriger Lösung auf 100 Gewichtsanteile des Materials imprägniert ist,
**dadurch gekennzeichnet,**
**dass** das Material ein natürlicher Zeolith ist, der wenigstens ein Material enthält, das unter Clinoptilotit, Mordenit, Chabazit, Erionit, Phillipsit, Heulandit und Analcim ausgewählt ist,
**dass** die wässerige Lösung auf 1000 Gewichtsanteilen Wasser zwischen ungefähr 1 Teil und ungefähr 20 Gewichtsanteilen einer verbundenen Mischung enthält, die auf einen Teil von Ammoniumionen oder eines Äquivalents, einen Teil wenigstens einer Zusammensetzung vom parfümierenden und/oder desodorierenden Typ enthält.

9. Verfahren nach Anspruch 8,
**dadurch gekennzeichnet,**
**dass** ein Zwangsumlauf der Atmosphäre oder des Gasstroms über das Filter durch Ansaugen der Atmosphäre oder des Gasstroms über das Filter erfolgt.

## Claims

1. Filtration device (1) for neutralizing the volatile unpleasant odors present in an atmosphere or conveyed by a stream of gas (5), this device comprising at least one layer (3) of adsorbent material (12) of determined thickness impregnated with an aqueous solution (14), with, per 100 parts by weight of material, about 5 to 20 parts by weight of said aqueous solution, **characterized in that**
the material is natural zeolite comprising at least one material chosen from clinoptilolite, mordenite, chabazite, erionite, phillipsite, heulandite and analcime,
**in that** the device comprises humidification means
and **in that** said aqueous solution is cationic and comprises, per 1000 parts by weight of water, between about 1 and 20 parts by weight of a composite mixture comprising, per one part of ammonium ions or equivalent, one part of at least one composition of perfuming and/or deodorizing nature.

2. Device according to Claim 1, **characterized in that** the aqueous solution comprises between about 3 and 10 parts of composite mixture.

3. Device according to either of the preceding claims, **characterized in that** the layer (3) made of natural zeolite is formed of granular material with an average particle size of between about 0.1 cm and about 5 cm.

4. Device according to any one of the preceding claims, **characterized in that** the composition of perfuming and/or deodorizing nature comprises an amount, which is effective for neutralizing the unpleasant odors, of a compound represented by the following structural formula: 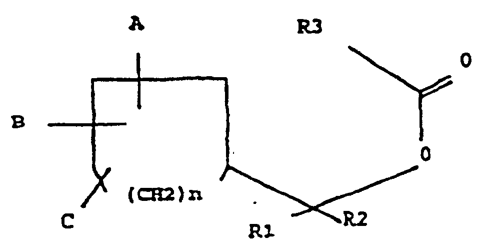 in which n is an integer from 2 to 4, A, B and C each independently represent hydrogen, a lower alkyl group containing 1 to 5 carbon atoms or a lower alkenyl group containing 3 to 5 carbon atoms, on condition that when two of the groups, A, B and C, are alkyl or alkenyl groups, they are attached to different carbon atoms and provided that the sum of the carbon atoms in A, B and C is not greater than 7, R1 and R2 each independently represent hydrogen or a lower alkyl group containing 1 to 5 carbon atoms, in which R1 and R2 together represent CH2m in which m is an integer from 2 to 6, R3 represents hydrogen or a lower alkyl or alkenyl group containing up to 6 carbon atoms, provided that the sum of the greatest number of carbon atoms in R1 or R2 plus R3 is not greater than 10.

5. Device according to any one of Claims 1 to 3, **characterized in that** the composition of perfuming and/or deodorizing nature is produced from aromatic esters and/or from a complex aldehyde formation.

6. Device according to Claim 5, **characterized in that** the perfuming composition comprises a first aldehyde chosen from acyclic and non-terpenic aliphatic aldehydes, alicyclic non-terpenic aldehydes, terpenic aldehydes, aliphatic aldehydes substituted with an aromatic group and biofunctional aldehydes and a second aldehyde chosen from aldehydes containing unsaturation α to the aldehyde function conjugated with an aromatic ring and aldehydes whose function is borne by an aromatic ring.

7. Device according to any one of the preceding claims, **characterized in that** it also comprises means for forced circulation of the stream of gas through the layer of material in the device.

8. Process for treating the volatile unpleasant odors present in an atmosphere or conveyed by a stream of gas, in which process the atmosphere or the stream of gas is treated by filtration with a filter composed of a layer of adsorbent material of determined thickness impregnated with an aqueous solution, with, per 100 parts by weight of material, about 5 to about 20 parts by weight of said aqueous solution, **characterized in that** the material is natural zeolite comprising at least one material chosen from clinoptilotite, mordenite, chabazite, erionite, phillipsite, heulandite and analcime,
said aqueous solution comprising, per 1000 parts by weight of water, between about 1 part and about 20 parts by weight of a composite mixture comprising, per one part of ammonium ions or equivalent, one part of at least one composition of perfuming and/or deodorizing nature.

9. Process according to Claim 8, **characterized in that** forced circulation of the atmosphere or of the stream of gas through the filter is carried out by sucking said atmosphere or said stream of gas through said filter.
